# EUROPÄISCHE PATENTSCHRIFT

(11) **EP 1 214 316 B1**
(45) Veröffentlichungstag und Bekanntmachung des Hinweises auf die Patenterteilung: **07.05.2003**
(21) Anmeldenummer: 00960572.6
(22) Anmeldetag: 31.08.2000
(51) Int. Cl.: C07D 409/12, C07D 405/12, C07D 333/28, C07D 307/54, C07D 413/12, C07D 417/12, C07D 239/26, C07D 239/34, C09K 19/04

(54) **FÜNFRING-VERBINDUNGEN UND IHRE VERWENDUNG IN FLÜSSIGKRISTALLINEN MISCHUNGEN**
FIVE MEMBERED-RING COMPOUNDS AND UTILIZATION THEREOF IN LIQUID CRYSTAL MIXTURES
COMPOSES A NOYAU PENTAGONAL ET LEUR UTILISATION DANS DES MELANGES MESOMORPHES

(30) Priorität: 01.09.1999 DE 19941649; 01.09.1999 DE 19941651; 01.09.1999 DE 19941650; 01.09.1999 DE 19941653; 01.09.1999 DE 19941654; 01.09.1999 DE 19941656
(43) Veröffentlichungstag der Anmeldung: 19.06.2002
(73) Patentinhaber: CLARIANT INTERNATIONAL LTD., 4132 Muttenz (CH)
(72) Erfinder: HORNUNG, Barbara, D-63594 Hasselroth (DE); NONAKA, Toshiaki, Shizuoka Pref. 436-0027 (JP); MATSUDA, Ayako, Machida-shi, Tokyo 194-0022 (JP); SCHMIDT, Wolfgang, D-63303 Dreieich (DE); WINGEN, Rainer, D-65795 Hattersheim (DE)
(74) Vertreter: Isenbruck, Günter, Dr.
(86) Internationale Anmeldenummer: EP0008518
(87) Internationale Veröffentlichungsnummer: WO01016131

(56) Entgegenhaltungen:
- EP-A- 0 364 923
- EP-A- 0 500 072
- DE-A- 4 446 836
- DE-A- 19 740 898
- GB-A- 2 229 179
- BROWN J W ET AL: "SOME THREE-RING ESTERS CONTAINING A FIVE-MEMBERED HETEROAROMATIC RING. A COMPARISON OF LIQUID CRYSTAL PROPERTIES" MOLECULAR CRYSTALS AND LIQUID CRYSTALS (INC. NONLINEAR OPTICS ),GB,GORDON AND BREACH SCIENCE PUBLISHERS, READING, Bd. 173, 1. August 1989 (1989-08-01), Seiten 121-140, XP000082009
- M.A. OSMAN ET AL: "stable liquid crystals with large negative dielectric anisotropy-III" MOLECULAR CRYSTALS AND LIQUID CRYSTALS, LETTERS , Bd. 82, 1983, Seiten 339-344, XP002161810
- D.F. ANDRES ET AL.: "reaction ofthioglycolate with alpha-Fluoro-beta-(phenylthio)enones (or -enals): Synthesis of substituted alpha-Carboxy-gamma-fluorothiophenes" TETRAHEDRON LETTERS, Bd. 38, Nr. 6, - 1997 Seiten 1049-1052, XP002154187 in der Anmeldung erwähnt
- C. CORRAL ET AL.: "the behaviour of vicinal alkyl aminothiophenecarboxylates in the sandmeyer and schiemann reactions" HETEROCYLES, Bd. 23, Nr. 6, - 1985 XP000972048 in der Anmeldung erwähnt
- DATABASE WPI Section Ch, Week 198817 Derwent Publications Ltd., London, GB; Class E13, AN 1988-115423 XP002161811 & JP 63 060981 A (KAWASAKI KASEI KOGYO KK) , 17. März 1988 (1988-03-17) in der Anmeldung erwähnt
- TAKENAKA S ET AL: "The Sa-Sa transition in a homologous series of 4(4-alkoxyphenoxycarbon" MOLECULAR CRYSTALS AND LIQUID CRYSTALS (INC. NONLINEAR OPTICS ),GB,GORDON AND BREACH SCIENCE PUBLISHERS, READING, Bd. 131, Nr. 3/04, 1985, Seiten 257-271, XP002134543

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z. B. EP-A 0 032 362). Aufgrund dieser und anderer günstiger Eigenschaften, z. B. der bistabilen Schaltmöglichkeit und des nahezu blickwinkelunabhängigen Kontrasts, sind FLCs grundsätzlich für Anwendungsgebiete wie Computerdisplays gut geeignet.

Für eine vertiefende Erörterung der technischen Anforderungen an FLCs wird auf die europäische Patentanmeldung 97118671.3 sowie die DE-A 197 48 432 verwiesen.

Für die Verwendung in Flüssigkristallmischungen sind bereits Thiophen-Derivate beschrieben, z.B. in EP-B 0 500 072. Einzelne 3- bzw. 4-Fluorthiophencarbonsäuren sind auch bereits beschrieben, z.B. inTetrahedron Letters 1997, 38(6), 1049; Heterocycles 23, 1431 (1985); Synth.Commun. 24, 95 (1994). Hinweise auf eine Eignung als Baustein für Flüssigkristalle lassen sich diesen letzten Schriften jedoch nicht entnehmen.

Für die Verwendung in nematischen Flüssigkristallmischungen sind Ester der Furanacrylsäure mit terminal-polaren Phenolen in JP-A 6306098 beschrieben.

Genannt seien in diesem Zusammenhang auch die folgenden Druckschriften: DE 4446836, J.W.BROWN et al. Mol. Cryst. Liqu. Cryst. 173, 121 (1989) sowie M. A. OSMAN et al. Mol. Cryst. Liqu. Cryst. 82, 339 (1983).

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert, unter anderem auch deshalb, weil erst das Zusammenwirken der flüssigkristallinen Mischungen mit den einzelnen Bauteilen der Anzeigevorrichtung bzw. der Zellen (z. B. der Orientierungsschicht) Rückschlüsse auf die Qualität auch der flüssigkristallinen Mischungen zuläßt.

Es wurde nun gefunden, daß Fünfring-Verbindungen der Formel (I) schon in geringen Zumischmengen die Eigenschaften von Flüssigkristallmischungen, insbesondere chiral-smektischen Mischungen, günstig beeinflussen, z. B. hinsichtlich der dielektrischen Anisotropie und/oder des Schmelzpunktes, aber auch hinsichtlich des Schaltverhaltens, den Werten des Tiltwinkels bzw. dessen Temperaturabhängigkeit.

Gegenstand der Erfindung sind daher Fünfring-Verbindungen der Formel (I),

R¹-X-(A¹-M¹)ₐ-(A²-M²)_{b}-A³-Y-E (I)

wobei die Symbole und Indizes folgende Bedeutungen haben:
- **E**: einen Fünfring enthaltender Rest T-Z-R² mit den Bedeutungen:
(i)
   **T** ungerichtet
   4-Fluorthiophen-2,5-diyl, 3-Fluorthiophen-2,5-diyl,
   3-Fluorthiophen-2,4-diyl oder 5-Fluorthiophen-2,4-diyl
   **Z** eine Einfachbindung oder -O-
   **R**^{**2**} Wasserstoff oder ein geradkettiger oder verzweigter Alkylrest (mit oder ohne asymmetrische C-Atome) mit 1 bis 20 C-Atomen, wobei eine nicht terminale CH₂-Gruppe durch -O- oder -OC(=O)- oder -C(=O)O- ersetzt sein kann und/oder ein oder mehrere H-Atome durch F ersetzt sein können mit den Maßgaben,
   a) daß die dem Thiophen nächste -CH₂-Gruppe dann nicht durch -O- ersetzt sein kann, wenn Z -O- ist
   b) R² nur dann Wasserstoff sein kann, wenn Z eine Einfachbindung ist,
(ii)
   **T** Furan-2,5-diyl oder Furan-2,4-diyl
   **Z** eine Einfachbindung oder -O-
   **R**^{**2**} ein geradkettiger oder verzweigter Alkylrest (mit oder ohne asymmetrische C-Atome) mit 1 bis 20 C-Atomen, wobei eine nicht terminale, nicht dem Furan benachbarte -CH₂-Gruppe durch -O- oder -OC(=O)- oder -C(=O)O- ersetzt sein kann und/oder ein oder mehrere H-Atome durch F ersetzt sein können,
- **R**^{**1**}: Wasserstoff oder ein geradkettiger oder verzweigter C₁₋₂₀-Alkyl- oder C₂₋₂₀-Alkenylrest (mit oder ohne asymmetrische C-Atome), wobei
a) eine oder zwei nicht terminale CH₂-Gruppen unabhängig voneinander durch -O- oder -C(=O)- ersetzt sein können mit der Maßgabe, daß zwei benachbarte CH₂-Gruppen nicht gleich ersetzt sein können und/oder
b) eine CH₂-Gruppe durch -C≡C- ersetzt sein kann und/oder
c) eine CH₂-Gruppe durch -Si(CH₃)₂-, Cyclopropan-1,2-diyl, Cyclobutan-1,3-diyl, Cyclopentan-1,4-diyl, Bicyclo[1.1.1]pentan-1,3-diyl oder Cyclohexan-1,4-diyl ersetzt sein kann und/oder
d) ein oder mehrere H-Atome durch F und/oder CN ersetzt sein können;
e) im Falle eines verzweigten Alkylrestes mit asymmetrischen C-Atomen die asymmetrischen C-Atome -CH₃, -OCH₃, -CF₃, F, CN und/oder Cl als Substituenten aufweisen oder
   in einen 3- bis 7-gliedrigen Ring eingebaut sind, worin auch eine oder zwei nicht benachbarte CH₂-Gruppen durch -O- und eine zu diesen nicht benachbarte CH₂-Gruppe durch -OC(=O)- ersetzt sein können;
- **X**: eine Einfachbindung, -O-, OC(=O)-, -C(=O)O- oder -OC(=O)O-
- **Y**: -OC(=O)-, -OCH₂-, -CH₂CH₂-
- **A**^{**1**}**, A**^{**2**}**, A**^{**3**}: sind unabhängig voneinander
Phenylen-1,4-diyl, gegebenenfalls ein- oder zweifach substituiert durch CN oder F, Phenylen-1,3-diyl, gegebenenfalls ein- oder zweifach substituiert durch CN oder F, Cyclohexan-1,4-diyl, wobei ein oder zwei H-Atome durch CN und/oder CH₃ und/oder F ersetzt sein können, 1-Cyclohexen-1,4-diyl, wobei ein H-Atom durch F ersetzt sein kann, 1-Alkyl-1-silacyclohexan-1,4-diyl, Pyridin-2,5-diyl, gegebenenfalls einfach substituiert durch F, Pyrimidin-2,5-diyl, gegebenenfalls einfach substituiert durch F, Cyclopentan-2,5-diyl, Thiophen-2,5-diyl;
- **M**^{**1**}**, M**^{**2**}: sind unabhängig voneinander ungerichtet
-OC(=O)-, -OCH₂-, -CH₂CH₂-, -OC(=O)CH₂CH₂-, -OCH₂CH₂CH₂-,
-C≡C-, -CH₂CH₂CH₂CH₂- oder eine Einfachbindung;
wenn **T** Furan-2,5-diyl oder Furan-2,4-diyl bedeutet, ist M¹ eine Einfachbindung;
**a, b** sind unabhängig voneinander gleich 0 oder 1.
"terminal" bedeutet z. B. in R¹ die an X oder an H anknüpfenden CH₂-Gruppen.
"ungerichtet" bedeutet die Möglichkeit eines spiegelverkehrten Einbaus der Gruppe.

Bei den Fünfring-Verbindungen der Formel (I) handelt es sich um fluorierte Thiophenderivate (i) oder Furanderivate (ii).

Gemäß einer Ausführungsform der Erfindung gelten eine oder mehrere der folgenden Maßgaben:
Für Thiophenderivate (i) sind A¹, A², A³ nicht Cyclopentan-2,5-diyl.
Für Furanderivate sind A¹, A², A³ nicht Cyclopentan-2,5-diyl.

### Fluorierte Thiophenderivate

Bevorzugte Thiophenderivate (i) sind die folgenden Verbindungen der Formeln (I-1) bis (I-33) 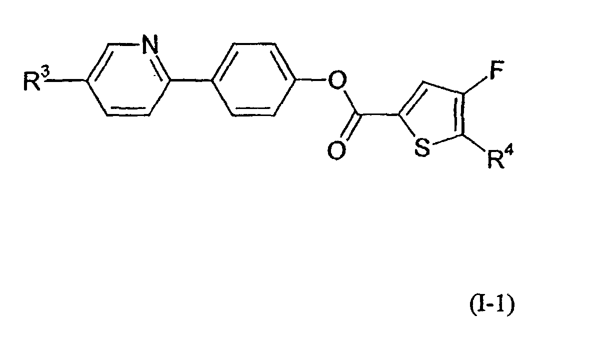 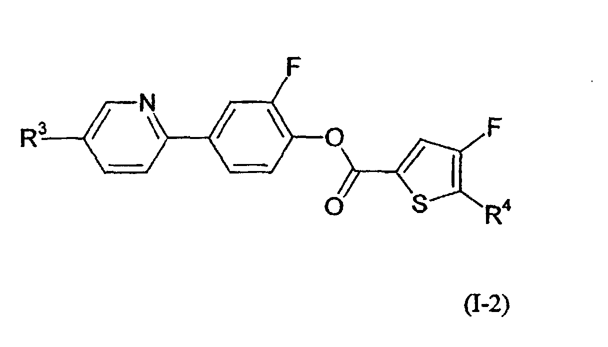 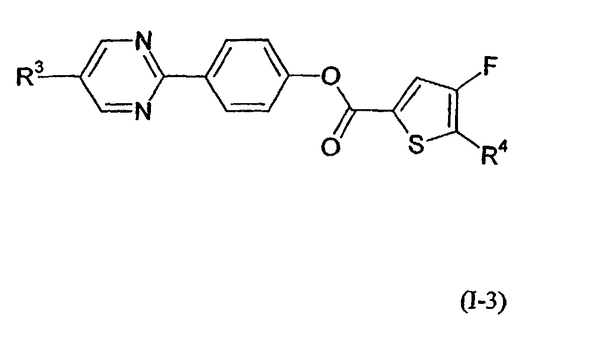 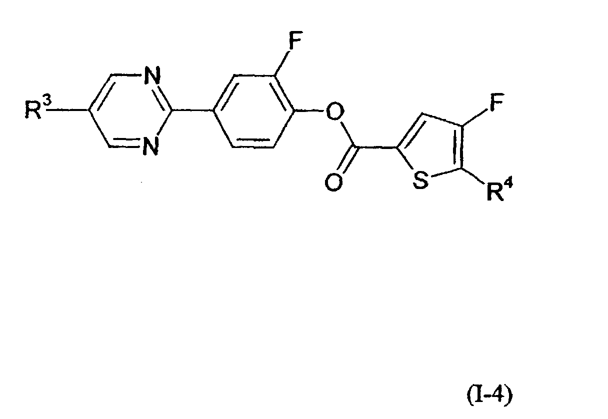 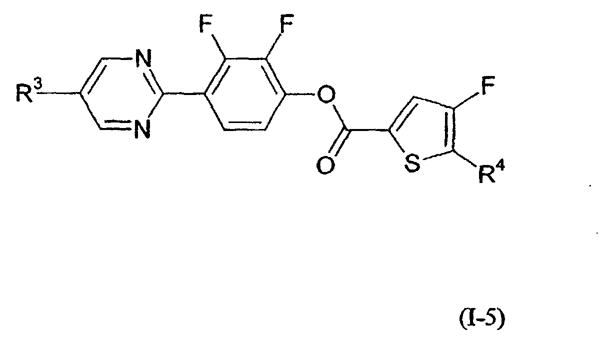 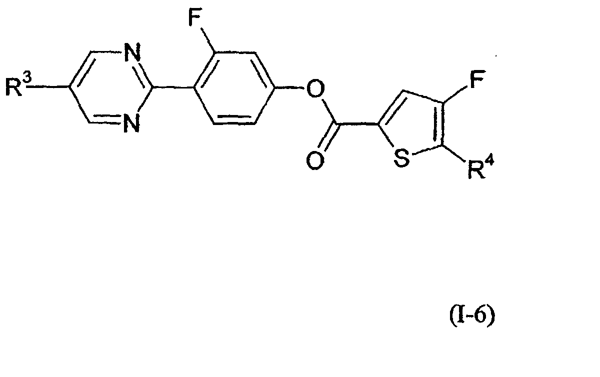 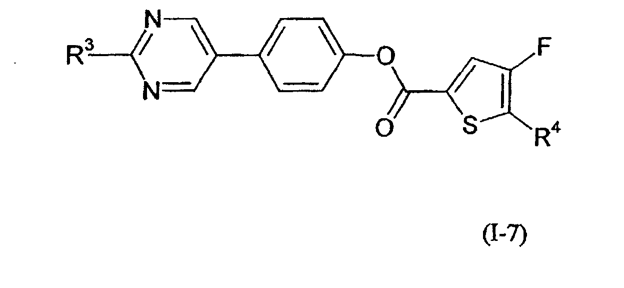 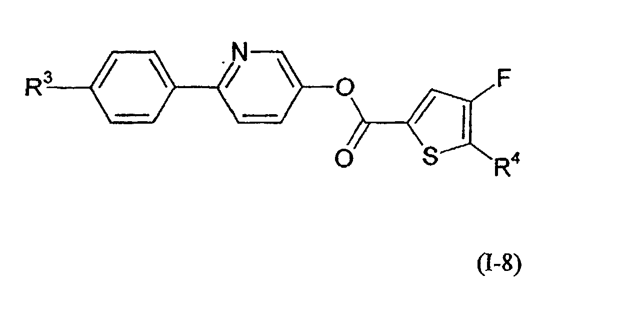 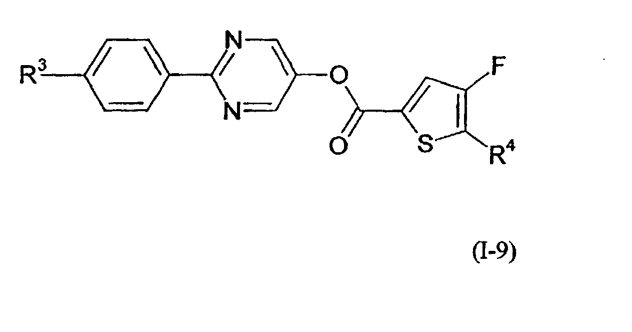 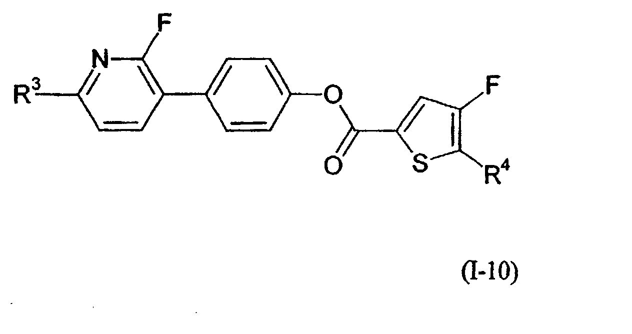 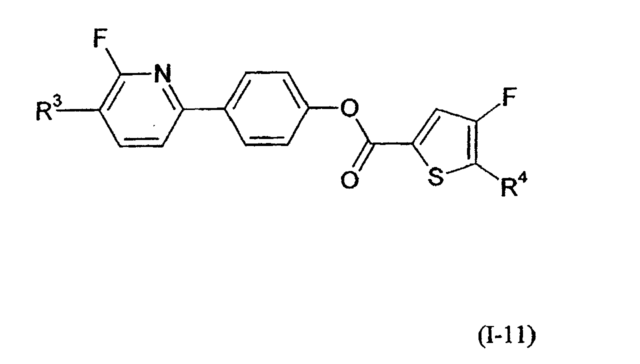 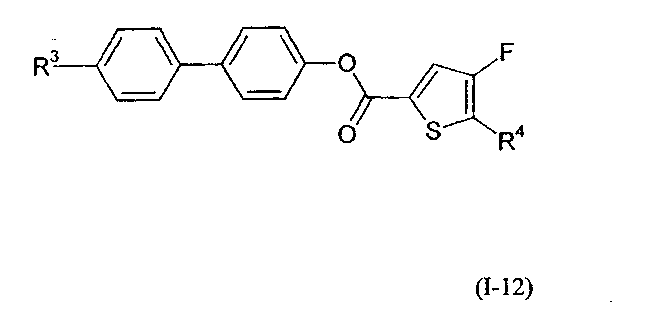 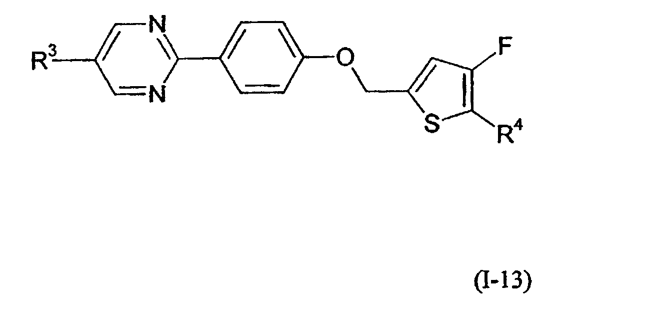 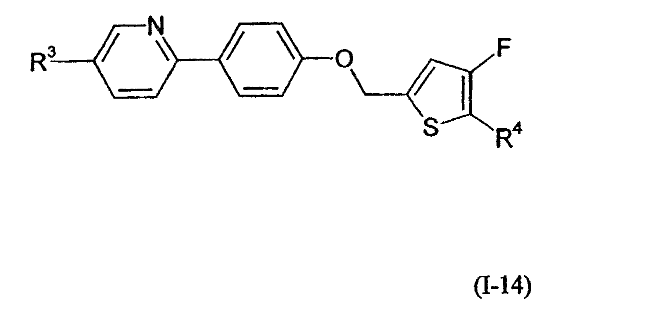 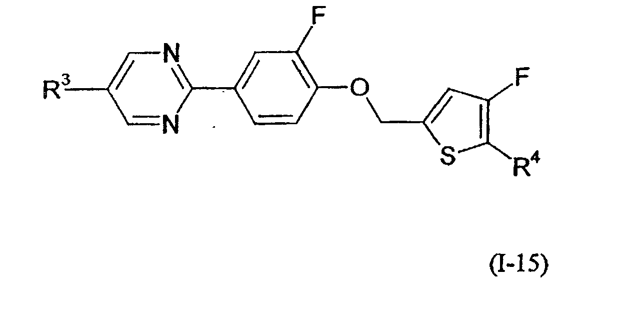 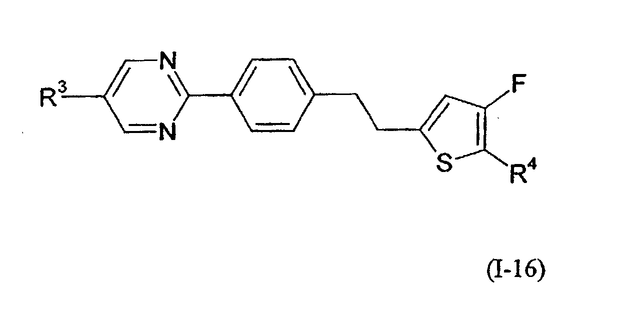 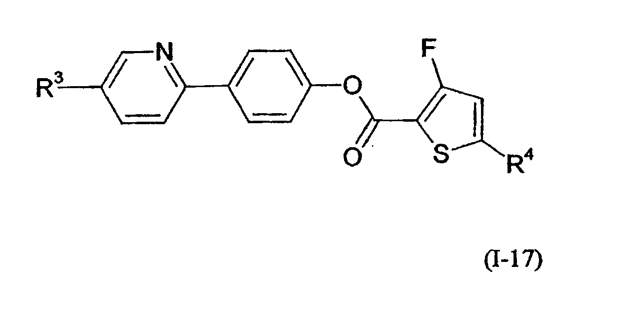 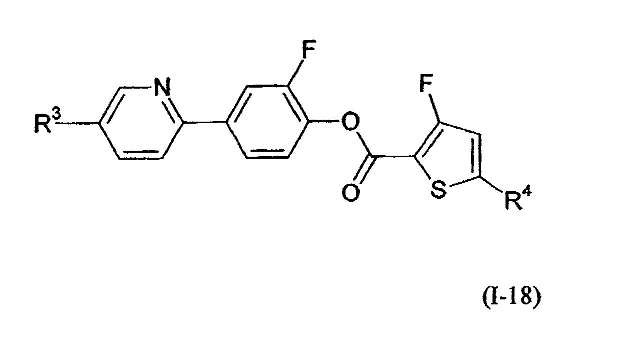 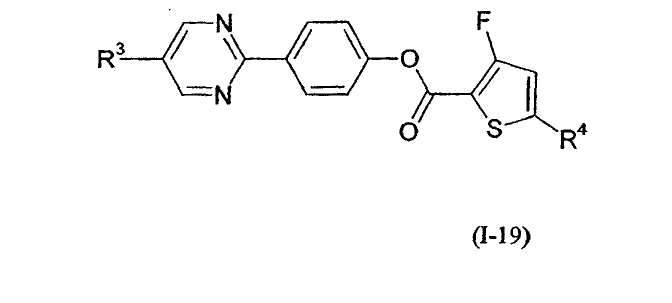 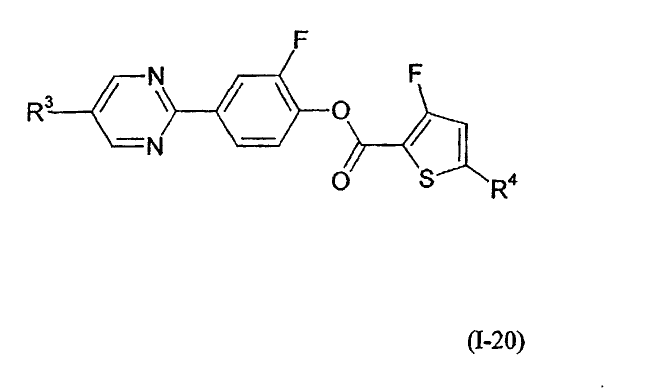 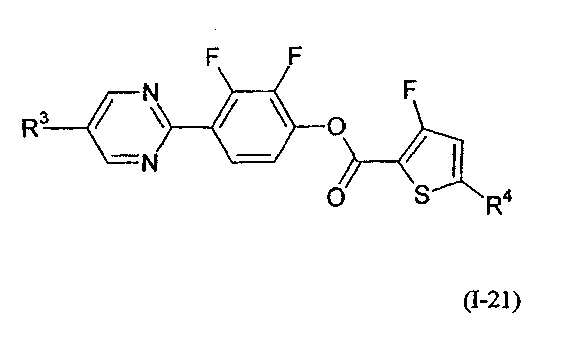 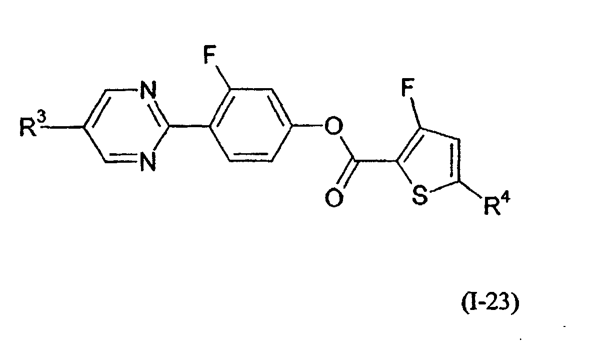 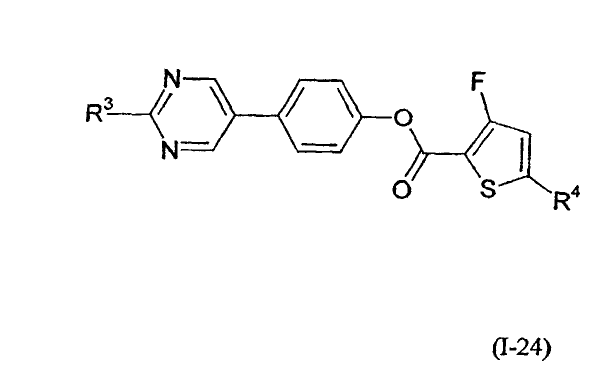 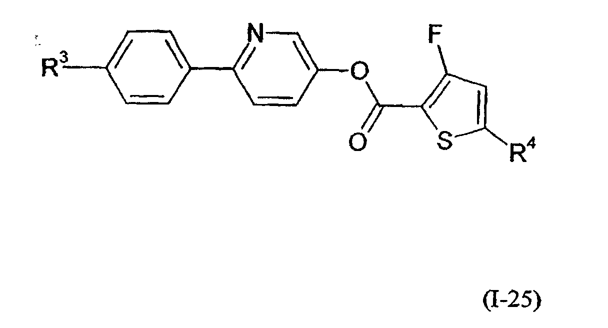 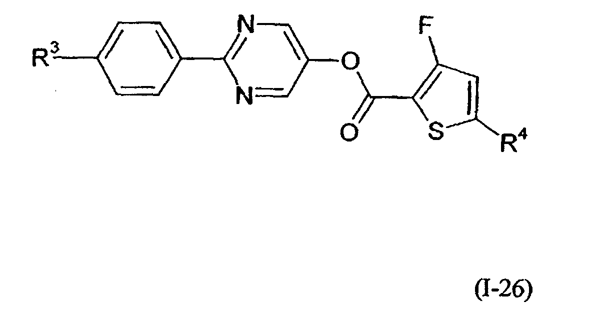 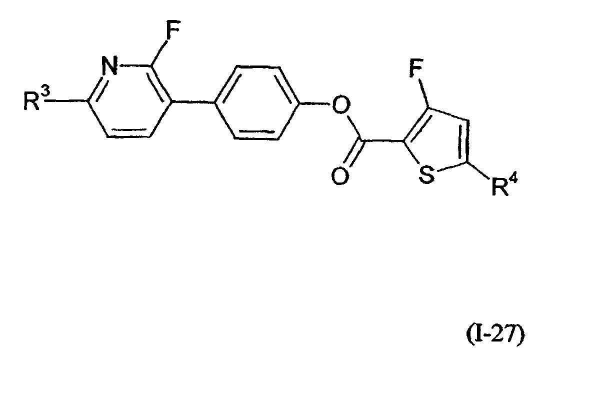 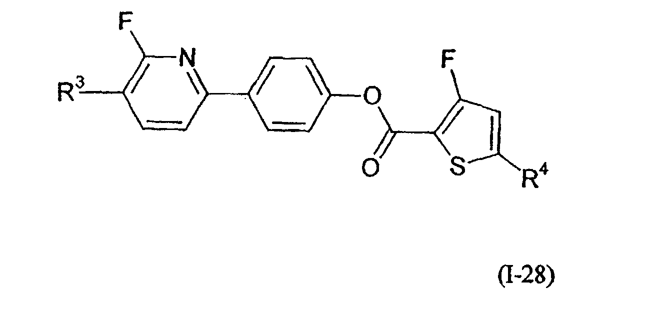 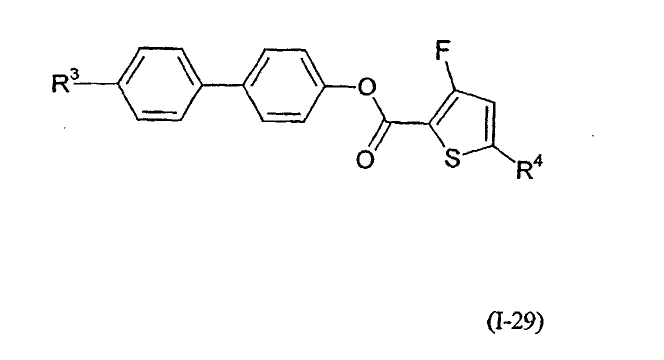 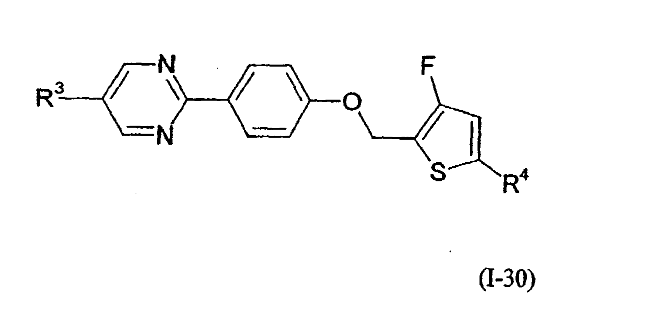 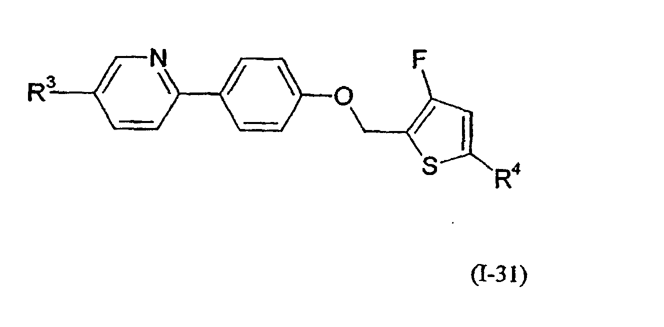 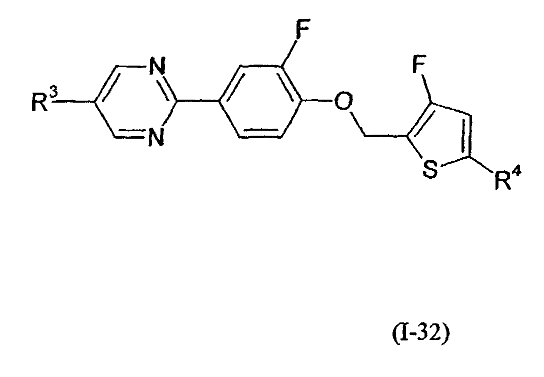 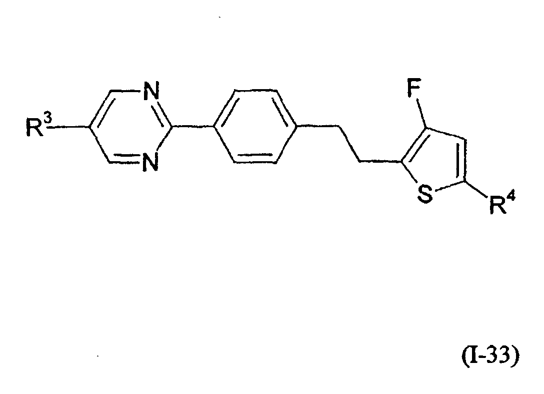 in denen bedeuten:
**R**^{**3**} Wasserstoff oder ein geradkettiger oder verzweigter Alkylrest (mit oder ohne asymmetrische C-Atome) mit 1 bis 16 C-Atomen, worin auch eine nicht terminale CH₂-Gruppe durch -O- oder ungerichtet -OC(=O)- ersetzt sein kann und worin ein oder mehrere H-Atome durch F ersetzt sein können;
**R**^{**4**} Wasserstoff oder ein geradkettiger oder verzweigter Alkylrest (mit oder ohne asymmetrische C-Atome) mit 1 bis 16 C-Atomen.

Besonders bevorzugt sind die Verbindungen der Formeln (I), insbesondere (I-1) bis (I-33), in denen **R**^{**3**} und **R**^{**4**} unabhängig voneinander einen geradkettigen Alkylrest mit 2 bis 16 C-Atomen bedeuten.

Ebenfalls besonders bevorzugt sind die Verbindungen der Formel (I), insbesondere (I-1) bis (I-33), in denen **R**^{**3**} einen geradkettigen Alkyloxyrest mit 2 bis 12 C-Atomen und **R**^{**4**} Wasserstoff oder einen geradkettigen Alkylrest mit 2 bis 12 C-Atomen bedeuten.

### Furanderivate

Bevorzugte Furanderivate (ii) sind die folgenden Verbindungen der Formeln (I-1) bis (I-16) 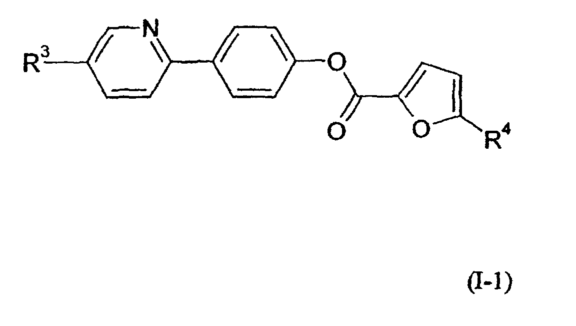 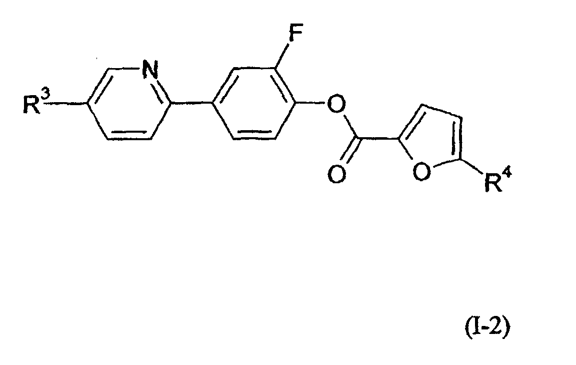 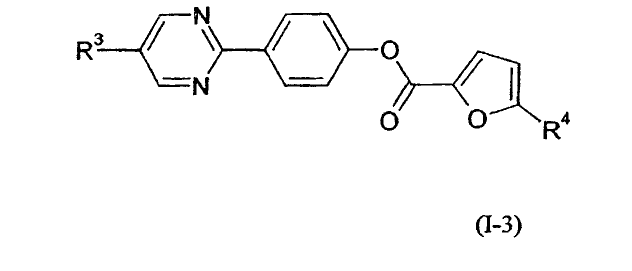 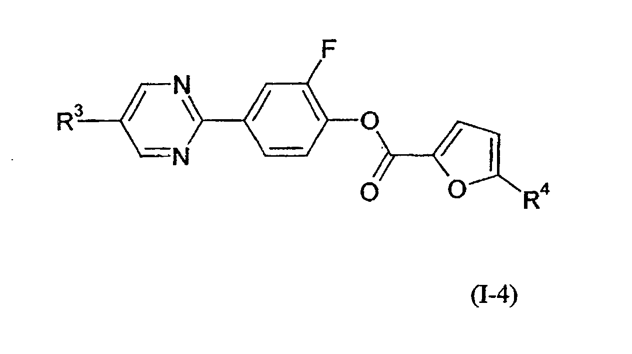 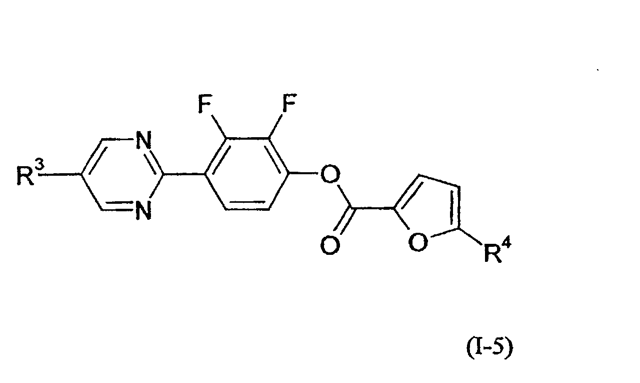 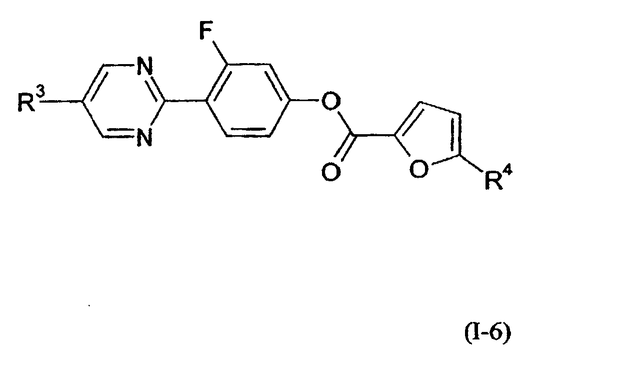 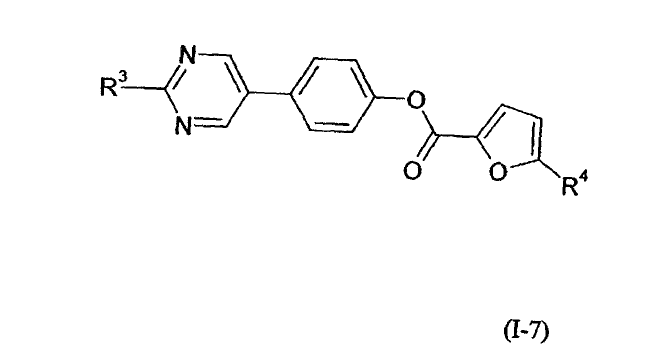 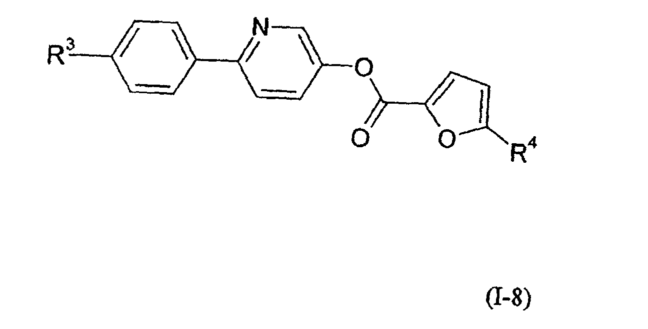 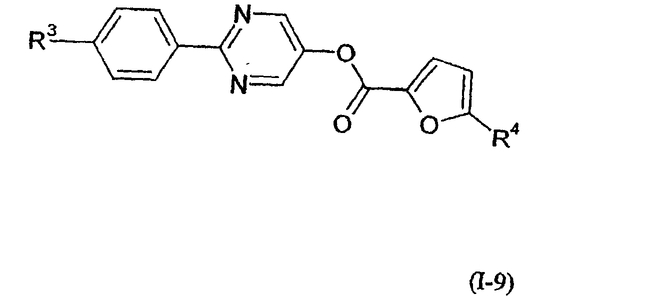 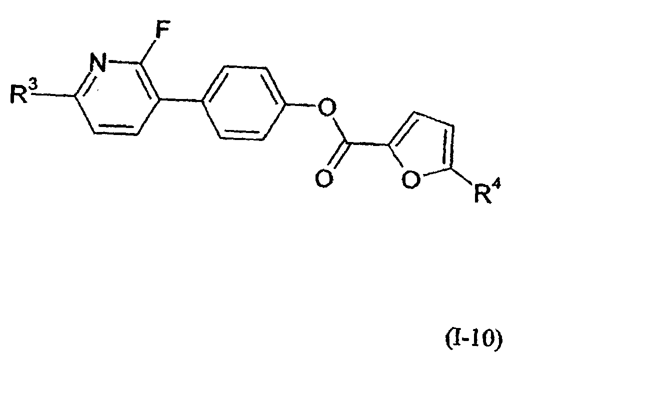 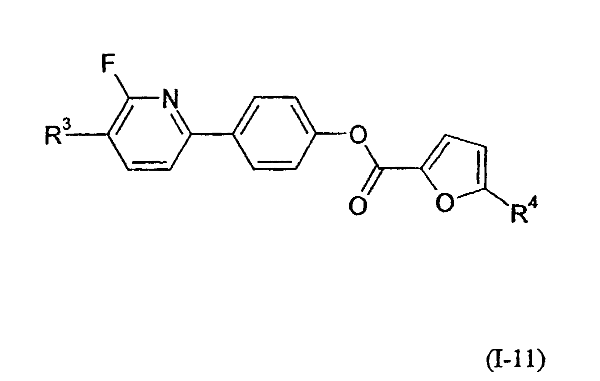 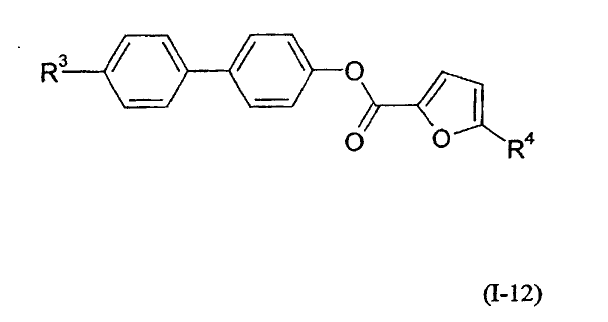 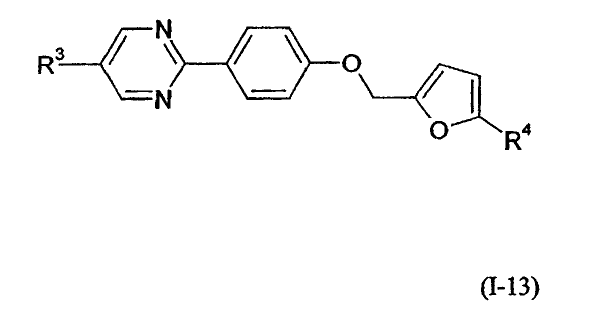 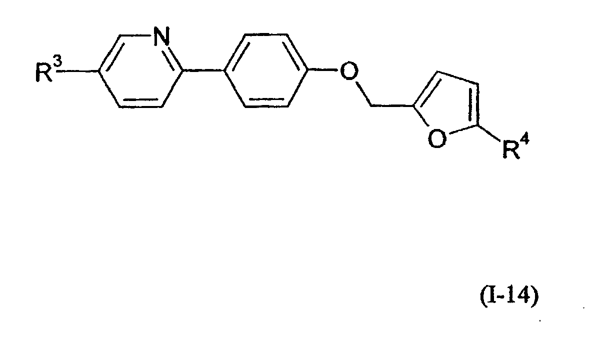 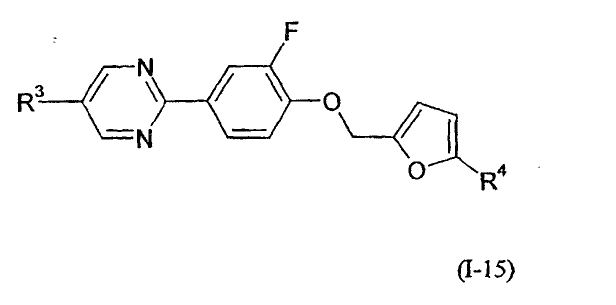 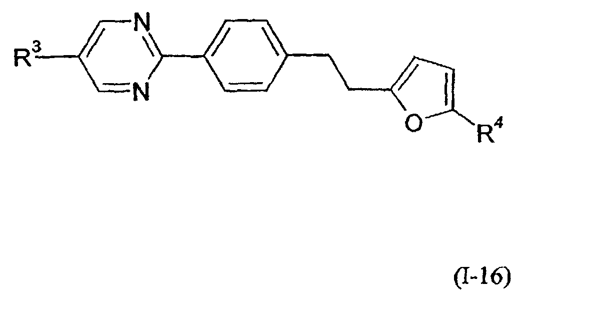 in denen bedeuten:
**R**^{**3**} Wasserstoff oder ein geradkettiger oder verzweigter Alkylrest (mit oder ohne asymmetrische C-Atome) mit 1 bis 16 C-Atomen, worin auch eine nicht terminale CH₂-Gruppe durch -O- oder ungerichtet -OC(=O)- ersetzt sein kann und worin ein oder mehrere H-Atome durch F ersetzt sein können;
**R**^{**4**} ein geradkettiger oder verzweigter Alkylrest (mit oder ohne asymmetrische C-Atome) mit 1 bis 16 C-Atomen.

Besonders bevorzugt sind die Verbindungen der Formeln (I), insbesondere (I-1) bis (I-16), in denen **R**^{**3**} und **R**^{**4**} unabhängig voneinander einen geradkettigen Alkylrest mit 2 bis 16 C-Atomen bedeuten.

Ebenfalls besonders bevorzugt sind die Verbindungen der Formeln (I), insbesondere (I-1) bis (1-16), in denen **R**^{**3**} einen geradkettigen Alkyloxyrest mit 2 bis 12 C-Atomen und **R**^{**4**} einen einen geradkettigen Alkylrest mit 2 bis 12 C-Atomen bedeuten.

Unter den Verbindungen der Formel (I), die als optisch aktive Komponenten (Dotierstoff) Einsatz in Flüssigkristallmischungen finden sollen, sind diejenigen bevorzugt, bei denen die Alkylgruppe die asymmetrischen C-Atome enthält in Form mindestens einer der Gruppierungen
a) -C*H(CH₃)CₘH₂ₘ₊₁, wobei m Werte von 2 bis 8 aufweist
b) -OC*H(CH₃)CₘH₂ₘ₊₁, wobei m Werte von 2 bis 8 aufweist
c) -OC*H(CH₃)CO₂CₘH₂ₘ₊₁, wobei m Werte von 1 bis 10 aufweist
d) -OC(=O)C*H(CH₃)OCₘH₂ₘ₊₁, wobei m Werte von 1 bis 10 aufweist
e) -OC(=O)C*H(F)CₘH₂ₘ₊₁, wobei m Werte von 1 bis 10 aufweist
f) -OCH₂C*H(F)CₘH₂ₘ₊₁, wobei m Werte von 1 bis 10 aufweist
g) -OCH₂C*H(F)C*H(F)CₘH₂ₘ₊₁, wobei m Werte von 1 bis 10 aufweist
h) Oxiran-2,3-diyl
worin C* das asymmetrische C-Atom markiert.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.

Es kann sich jedoch als erforderlich erweisen, die Literaturmethoden für die Erfordernisse mesogener Bausteine zu variieren / modifizieren, da z. B. funktionelle Derivate mit langen (> C₆) Alkylketten häufig ein geringeres Reaktionsvermögen zeigen als z. B. die Methyl- oder Ethylanaloga.

Insbesondere wird in diesem Zusammenhang für Thiophenderivate (i) auf nachstehende Syntheseschemata verwiesen, in denen die Synthese der erfindungsgemäßen Thiophen-Derivate beispielhaft näher erläutert wird. 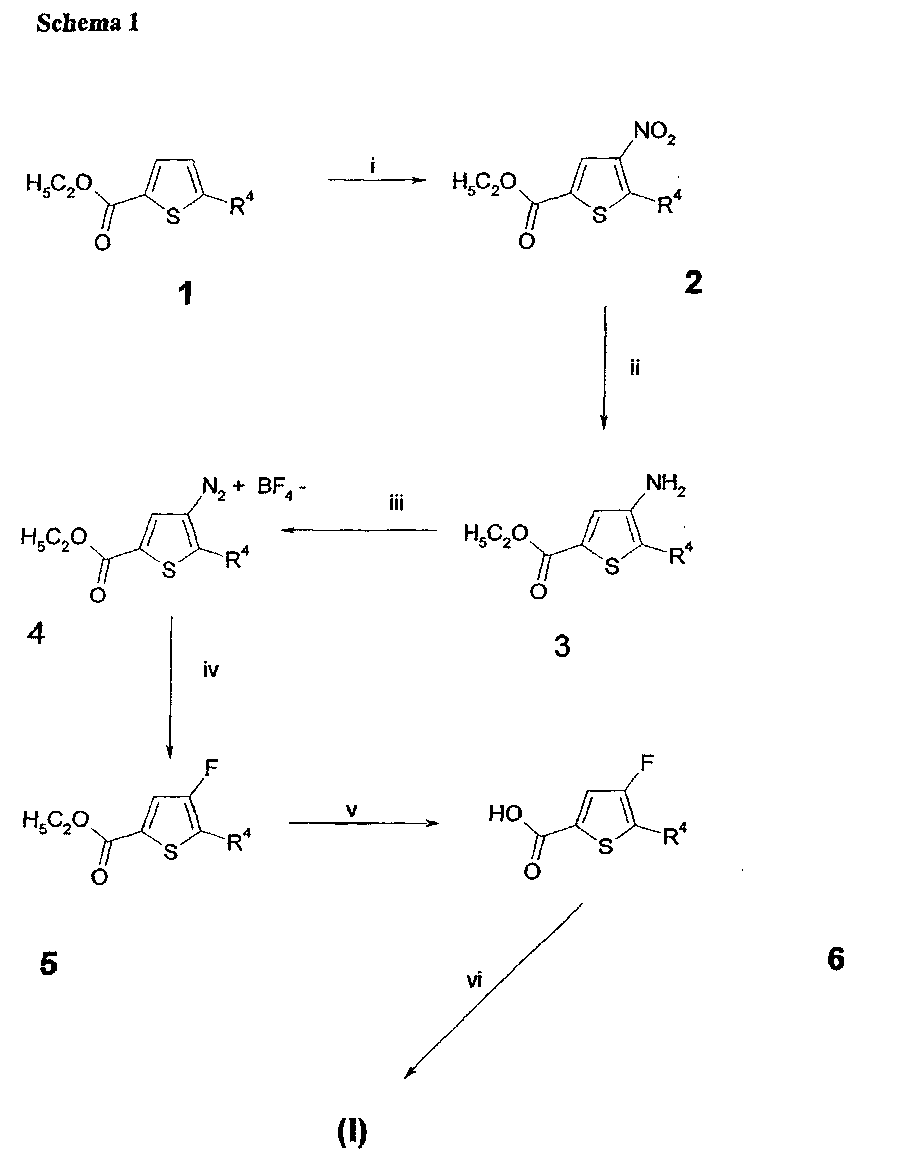
i: HNO₃, H₂SO₄ analog Campaigne, J.Am.Chem.Soc. 73, 3812 (1951).
ii: Sn, HCI analog Dewar, J.Am.Chem.Soc. 84, 3782 (1962)
iii: 1. NaNO₂ 2. HBF₄ analog Corral, Hetereocycles 23, 1431 (1985)
iv: Thermolyse analog Corral, Heterocycles 23, 1431 (1985)
v: 1. NaOH, EtOH 2. H⁺ analog Corral, Heterocycles 23, 1431 (1985)
vi: R¹-X-(A¹-M¹)ₐ-(A²-M²)_{b}-A³-OH, DCC / CH₂Cl₂

Dabei werden die für die Synthese nach Schema 1 benötigten 5-Alkylthiophen-2-carbonsäureester **1** analog EP-B 0 500 072 hergestellt. 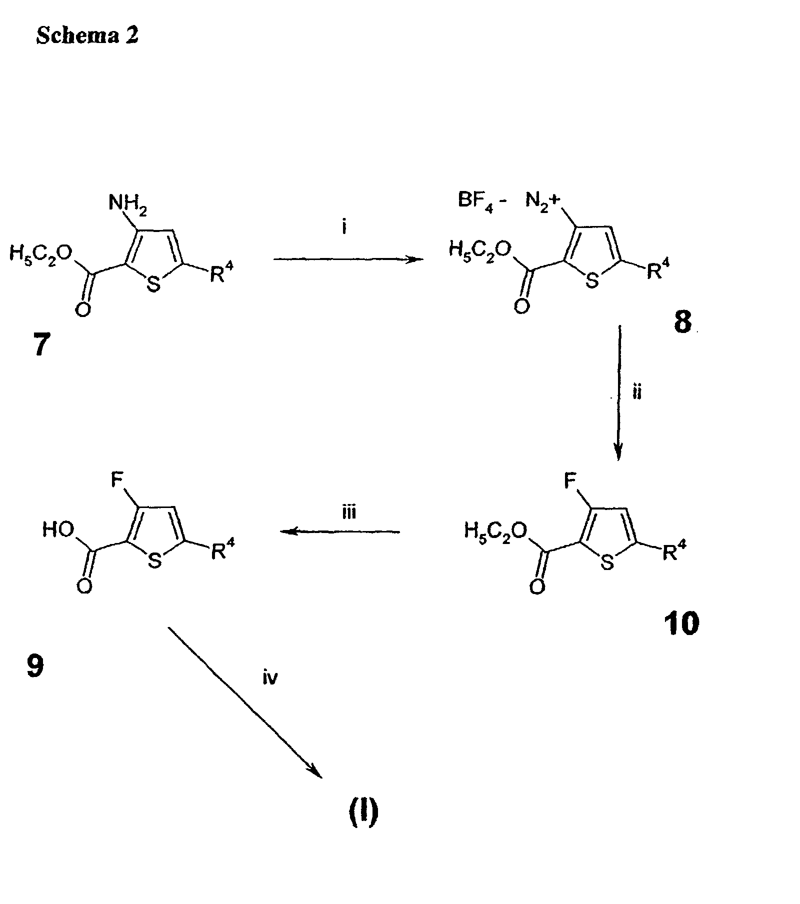
i: 1. NaNO₂ 2. HBF₄ analog Corral, Hetereocycles 23, 1431 (1985)
ii: Thermolyse analog Corral, Heterocycles 23, 1431 (1985)
iii: 1. NaOH, EtOH 2. H⁺ analog Corral, Heterocycles 23, 1431 (1985)
iv: R¹-X-(A¹-M¹)ₐ-(A²-M²)_{b}-A³-OH, DCC / CH₂Cl₂

Dabei werden die für die Synthese nach Schema 2 benötigten 5-Alkyl-3-aminothiophen-2-carbonsäureester 7 analog Huddleston, Synth.Commun. 9, 731 (1979) oder JP-A 05117263 oder JP-A 06025221 hergestellt. 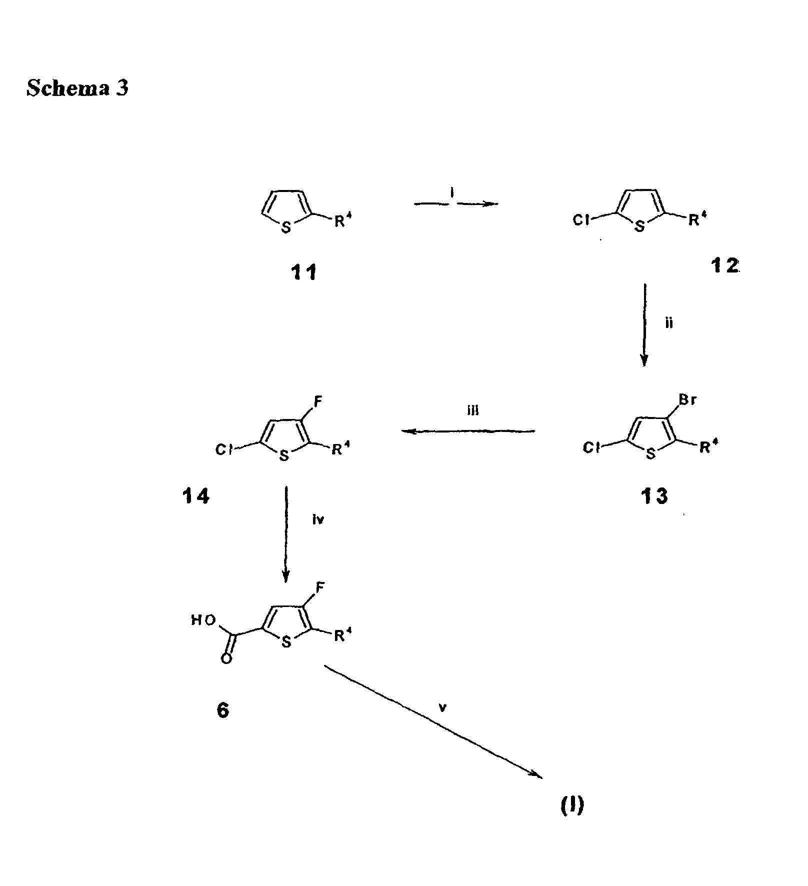
i: N-Chlorsuccinimid, HOAc, Benzol analog Lucas, Tetrahedron Lett. 40, 1775 (1999)
ii: Br₂, CHCl₃ analog Lucas, Tetrahedron Lett. 40, 1775 (1999)
iii: 1. BuLi 2. F-TEDA-BF₄ 1.Teilschritt analog Lucas, Tetrahedron Lett. 40, 1775 (1999)
iv: 1. BuLi 2. CO₂ analog Lucas, Tetrahedron Lett. 40, 1775 (1999)
v: R¹-X-(A¹-M¹)ₐ-(A²-M²)_{b} - A³ -OH, DCC / CH₂Cl₂

Die Erfindung betrifft auch die als Zwischenprodukte erhaltenen
5-Alkyl-4-fluor-thiophen-2-carbonsäuren der Formel (II) 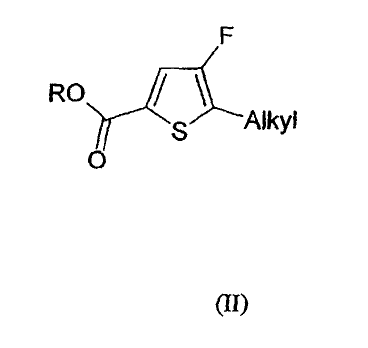 in der Alkyl ein geradkettiger oder verzweigter Alkylrest von 2 bis 16 C-Atomen, ausgenommen tert.-Butyl, und R Wasserstoff, Alkalimetall, Erdalkalimetall (1/2), ein geradkettiger oder verzweigter Alkylrest von 1 bis 16 Atomen, ausgenommen Methyl und tert.-Butyl ist und
5-Alkyl-3-fluor-thiophen-2-carbonsäuren der Formel (III) 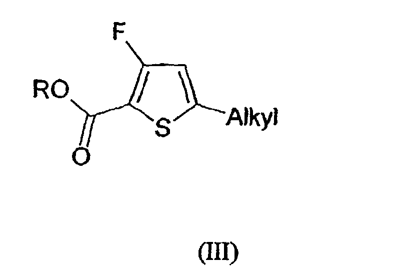 in der Alkyl ein geradkettiger oder verzweigter Alkylrest von 2 bis 16 C-Atomen und R Wasserstoff, Alkalimetall, Erdalkalimetall (1/2), ein geradkettiger oder verzweigter Alkylrest von 1 bis 16 Atomen ist, und entsprechende Säurehalogenide, insbesondere Säurechloride davon.

Sie entsprechen teilweise den vorstehenden Verbindungen der Formeln 5, 6, 9 und 10. Sie können für die Herstellung von Flüssigkristallen eingesetzt werden.

Die für die Synthese nach Schema 3 benötigten 2-Alkylthiophene **11** können nach EP-B 0 500 072 erhalten werden.

Was die Verknüpfung funktioneller Derivate der Furane und fluorierten Thiophene mit anderen flüssigkristallspezifischen Bausteinen anbelangt, wird ausdrücklich auf DE-A 197 48 432 verwiesen, in der eine Auflistung dem Fachmann geläufiger Methoden angegeben ist.

Gegenstand der Erfindung ist auch die Verwendung von Verbindungen der Formel (I) in Flüssigkristallmischungen, vorzugsweise smektischen und nematischen, besonders bevorzugt chiral-smektischen (ferroelektrischen) Flüssigkristallmischungen. Insbesondere bevorzugt ist die Verwendung in ferroelektrischen Flüssigkristallmischungen, die im Inverse-Mode oder in Anzeigen mit Aktivmatrix-Elementen betrieben werden.

Ganz besonders bevorzugt ist die Verwendung in Mischungen für Aktivmatrix-LCDs, bei denen die chiral-smektische Flüssigkristallschicht eine monostabile Monodomäne ausbildet

Weiterhin Gegenstand der Erfindung sind Flüssigkristallmischungen, vorzugsweise smektische und nematische, besonders bevorzugt ferroelektrische (chiral smektische), enthaltend eine oder mehrere Verbindungen der Formel (I).

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, bezogen auf die gesamte Mischung, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. In diesem Sinne geeignete weitere Mischungskomponenten sind insbesondere in der internationalen Patentanmeldung PCT/EP 96/03154 sowie DE-A 197 48 432 aufgeführt, auf die hiermit ausdrücklich Bezug genommen wird.

Die erfindungsgemäßen Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Ein weiterer Gegenstand der Erfindung ist daher eine Schalt- und/oder Anzeigevorrichtung, enthaltend eine, vorzugsweise smektische, Flüssigkristallmischung, die eine oder mehrere Verbindungen der Formel (I) enthält

Insbesondere bevorzugt sind ferroelektrische Schalt- und/oder Anzeigevorrichtungen, die Aktivmatrix-Elemente enthalten (siehe z. B. DE-A 198 22 830).

In der vorliegenden Anmeldung sind verschiedene Dokumente zitiert, beispielsweise um das technische Umfeld der Erfindung zu illustrieren.

Die Erfindung wird durch die nachfolgenden Beispiele weiter erläutert, ohne sie dadurch beschränken zu wollen.

### Thiophenderivate

### Beispiel 1

### 4-Fluor-5-propyl-thiophen-2-carbonsäure-[4-(5-undecyl-pyrimidin-2-yl)phenyl]ester

4,9 g 4 - (5-Undecyl-pyrimidin-2-yl)phenol, 2,0 g 4-Fluor-5-propyl-thiophen-2-carbonsäure (hergestellt nach Schema 1 durch Nitrierung von 5-Propyl-thiophen-2-carbonsäuremethylester in HNO₃ / H₂SO₄ zu 4-Nitro-5-propyl-thiophen-2-carbonsäuremethylester, Reduktion desselben zur entsprechenden Aminoverbindung mittels Sn / HCl, Überführung ins Diazoniumtetrafluoroborat, dessen Thermolyse und schließlich Verseifung) und 2,1 g Dicyclohexylcarbodiimid werden in 50 ml Dichlormethan 24 h bei Raumtemperatur gerührt. Nach Filtration, Abdestillation des Dichlormethans, chromatografischer Reinigung (Kieselgel; Dichlormethan / Heptan) und Umkristallisation aus Acetonitril wird die Zielverbindung als farblose Kristalle erhalten.

Analog können die Verbindungen (I-1) bis (I-12) erhalten werden bzw. unter Verwendung der nach Schema 2 hergestellten 5-Alkyl-3-fluor-thiophen-2-carbonsäuren die Verbindungen (I-17) bis (I-29).

### Beispiel 2

### (4-Fluor-5-propyl-thiophen-2-yl)methyl-[4-(5-undecyl-pyrimidin-2-yl)phenyl]ether

Zu einer abreagierten Mischung äquimolarer Mengen Diethylazodicarboxylat und Triphenylphosphan in THF werden äquimolare Mengen 4 - (5-Undecylpyrimidin-2-yl)phenol und 4-Fluor-5-propyl-thiophen-2-yl-methanol (hergestellt durch LiAlH₄-Reduktion von 4-Fluor-5-propyl-thiophen-2-carbonsäuremethylester) gegeben. Nach 24 h bei Raumtemperatur wird im Vakuum zur Trockne gebracht. Nach chromatografischer Reinigung (Kieselgel, Dichlormethan) und Umkristallisation kann die Zielverbindung erhalten werden.

Analog können die Verbindungen (I-13) bis (I-15) sowie (I-30) bis (I-32) erhalten werden.

Die Verbindungen der Formeln (I-16) bzw. (I-33) können über die Sequenz 4-(bzw. 3-)Fluor-5-alkyl-thiophen-2-yl-methanol - 2-Brommethyl-4-(bzw. 3-) fluor-5-alkyl-thiophen -- 2-Brommethyl-5-alkyl-4-(bzw. 3-)fluor-thiophen-2-yltriphenylphosphoniumsalz -- Wittig-Reaktion mit 4-(5-R³-pyrimidin-2-yl)benzaldehyd -- Hydrierung erhalten werden.

### Furanderivate

### Beispiel 1

### 5-Ethyl-furan-2-carbonsäure-[4-(5-undecyl-pyrimidin-2-yl)phenyl]ester

4,9 g 4 - (5-Undecyl-pyrimidin-2-yl)phenol, 1,4 g 5-Ethyl-2-furancarbonsäure (hergestellt nach Perry et al., Appl.Organomet.Chem. 10, 389-392 (1996) aus Furan-2-carbonsäure; Schmp. 90°C) und 2,1 g Dicyclohexylcarbodiimid werden in 50 ml Dichlormethan 24 h bei Raumtemperatur gerührt. Nach Filtration, Abdestillation des Dichlormethans, chromatografischer Reinigung (Kieselgel; Dichlormethan / Heptan) und Umkristallisation aus Acetonitril wird die Zielverbindung als farblose Kristatle mit der Phasenfolge X 80 (N62) I erhalten.

Analog wird erhalten

### Beispiel 2

### 5-Ethyl-furan-2-carbonsäure-[2-fluor-4-(5-undecyl-pyridin-2-yl)phenyl]ester mit Schmelzpunkt 76°C.

Analog Beispiel 1 lassen sich die Verbindungen (I-1) bis (I-12) herstellen.

### Beispiel 3

### (5-Ethyl-furan-2-yl)methyl-[4-(5-undecyl-pyrimidin-2-yl)phenyl]ether

Zu einer abreagierten Mischung äquimolarer Mengen Diethylazodicarboxylat und Triphenylphosphan in THF werden äquimolare Mengen 4,9 g 4 - (5-Undecylpyrimidin-2-yl)phenol und 5-Ethyl-furan-2-yl-methanol (hergestellt durch LiAlH₄-Reduktion von 5-Ethyl-furan-2-carbonsäuremethylester, der seinerseits durch Veresterung der 5-Ethyl-furan-2-carbonsäure aus Beispiel 1 erhältlich ist) gegeben. Nach 24 h bei Raumtemperatur wird im Vakuum zur Trockne gebracht.

Nach chromatografischer Reinung (Kieselgel, Dichlormethan) und Umkristallisation kann die Zielverbindung erhalten werden.

Analog können die Verbindungen (I-13) bis (I-15) erhalten werden.

Die Verbindungen der Formel (I-16) können über die Sequenz 5-Ethyl-furan-2-ylmethanol -- 2-Brommethyl-5-ethyl-furan --2-Brommethyl-5-ethyl-furan-triphenylphosphoniumsalz - Wittig-Reaktion mit 4-(5-R³-pyrimidin-2-yl)benzaldehyd -- Hydrierung erhalten werden.

Analog wurden die folgenden Verbindungen erhalten:

### Beispiel 4

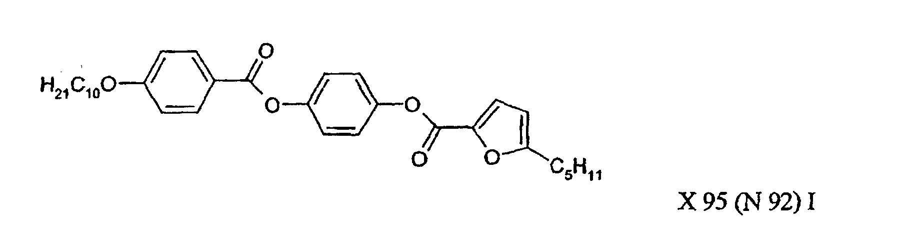

5-Pentylfuran-2-carbonsäure-[4-(4-decyloxy-benzoyloxy)phenyl]ester

### Beispiel 5

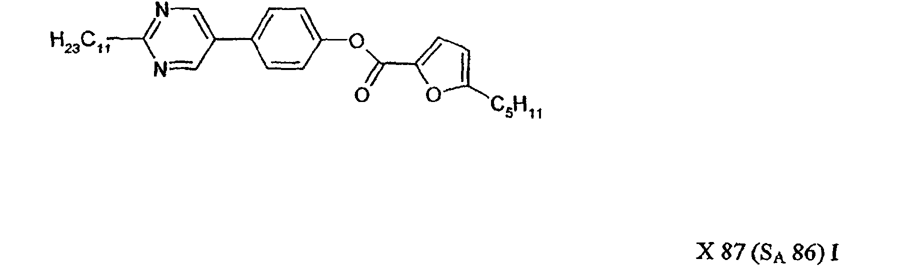

5-Pentylfuran-2-carbonsäure-[4-(2-undecyl-pyrimidin-5-yl)phenyl]ester

### Beispiel 6

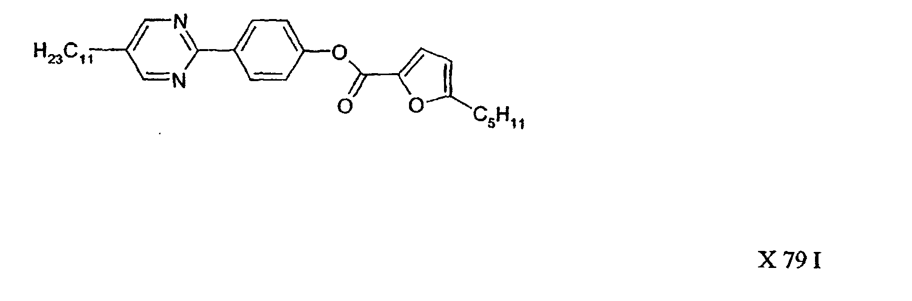

5-Pentylfuran-2-carbonsäure-[4-(5-undecyl-pyrimidin-2-yl)phenyl]ester

### Beispiel 7

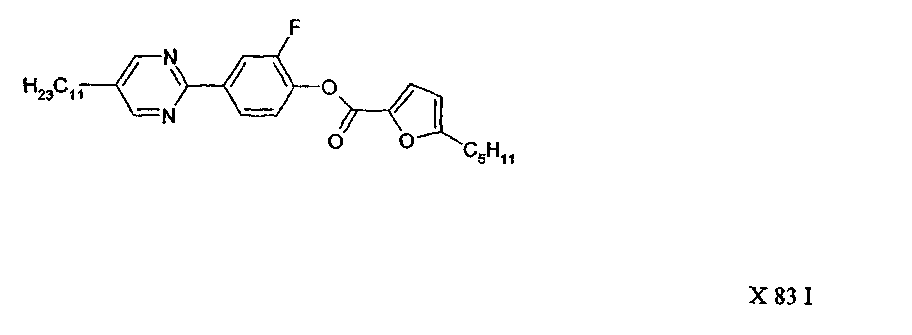

5-Pentylfuran-2-carbonsäure-[2-fluor-4-(5-undecyl-pyrimidin-2-yl)phenyl]ester

### Beispiel 8

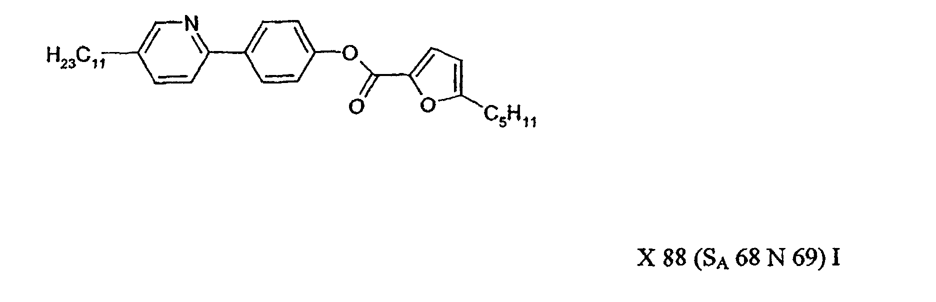

5-Pentylfuran-2-carbonsäure-[4-(5-undecyl-pyridin-2-yl)phenyl]ester

### Beispiel 9

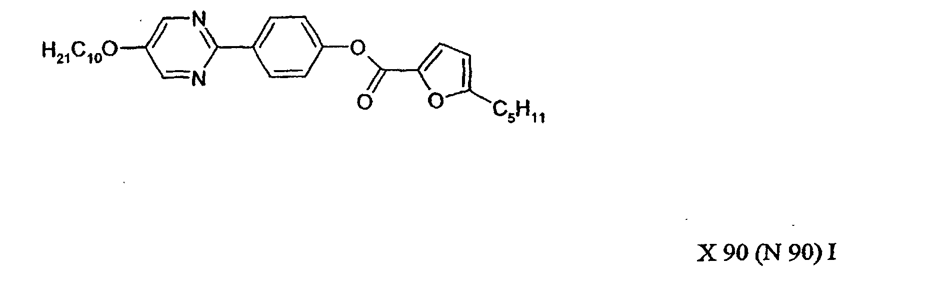

5-Pentylfuran-2-carbonsäure-[4-(5-decyloxy-pyrimidin-2-yl)phenyl]ester

### Beispiel 10

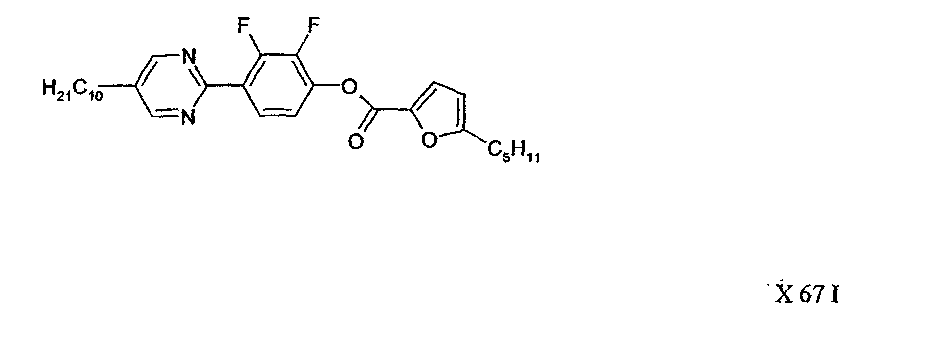

5-Pentylfuran-2-carbonsäure-[2,3-difluor-4-(5-decyl-pyrimidin-2-yl)phenyl]ester

### Beispiel 11

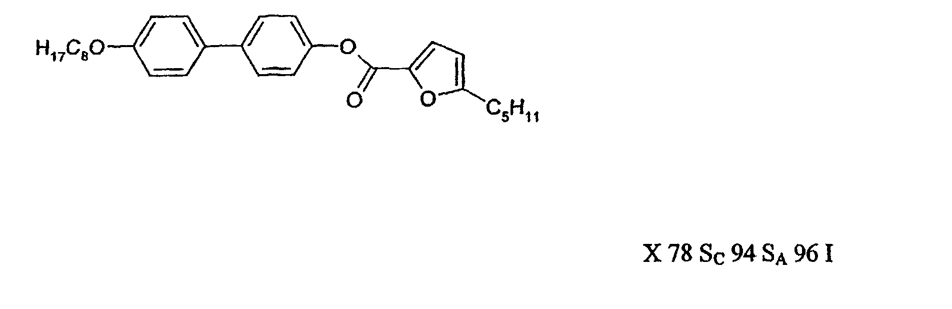

5-Pentylfuran-2-carbonsäure-[4-(4-octyloxyphenyl)phenyl]ester

### Beispiel 12

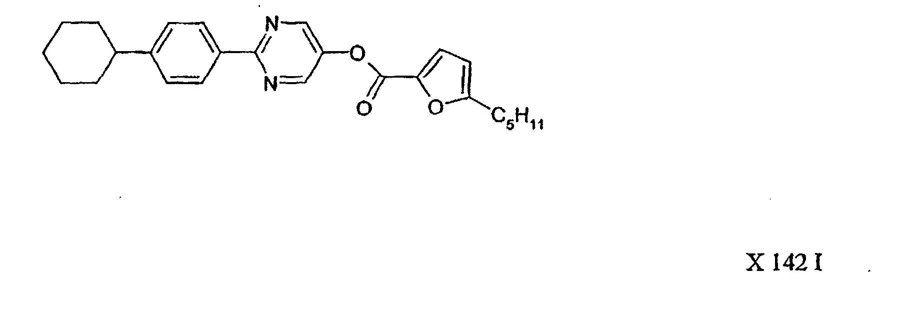

5-Pentylfuran-2-carbonsäure-[2-(4-cyclohexylphenyl)pyrimidin-5-yl]ester

### Beispiel 13

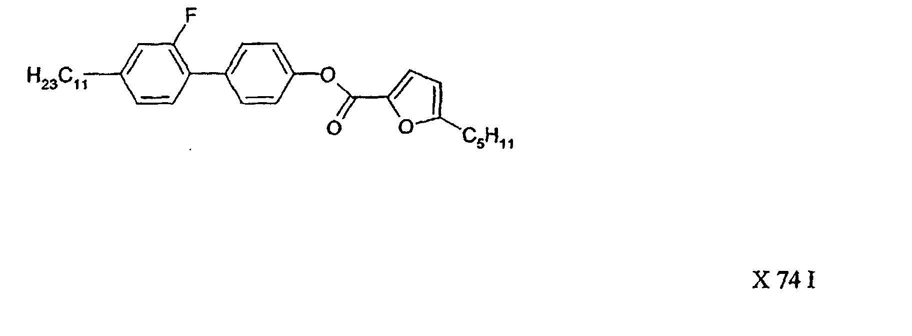

5-Pentylfuran-2-carbonsäure-[4-(2-fluor-4-undecyl-phenyl)phenyl]ester

## Patentansprüche

1. Fünfring-Verbindungen der Formel (I),
R¹-X-(A¹-M¹)ₐ-(A²-M²)_{b}-A³-Y-E (I)
wobei die Symbole und Indizes folgende Bedeutungen haben:
**E** einen Fünfring enthaltender Rest T-Z-R² mit den Bedeutungen:
(i)
**T** ungerichtet
4-Fluorthiophen-2,5-diyl, 3-Fluorthiophen-2,5-diyl,
3-Fluorthiophen-2,4-diyl oder 5-Fluorthiophen-2,4-diyl
**Z** eine Einfachbindung oder -O-
**R**^{**2**} Wasserstoff oder ein geradkettiger oder verzweigter Alkylrest (mit oder ohne asymmetrische C-Atome) mit 1 bis 20 C-Atomen, wobei eine nicht terminale CH₂-Gruppe durch -O- oder -OC(=O)- oder -C(=O)O- ersetzt sein kann und/oder ein oder mehrere H-Atome durch F ersetzt sein können mit den Maßgaben,
a) daß die dem Thiophen nächste -CH₂-Gruppe dann nicht durch -O- ersetzt sein kann, wenn Z -O- ist
b) R² nur dann Wasserstoff sein kann, wenn Z eine Einfachbindung ist,
(ii)
**T** Furan-2,5-diyl oder Furan-2,4-diyl
**Z** eine Einfachbindung oder -O-
**R**^{**2**} ein geradkettiger oder verzweigter Alkylrest (mit oder ohne asymmetrische C-Atome) mit 1 bis 20 C-Atomen, wobei eine nicht terminale, nicht dem Furan benachbarte -CH₂-Gruppe durch -O- oder -OC(=O)- oder -C(=O)O- ersetzt sein kann und/oder ein oder mehrere H-Atome durch F ersetzt sein können,
**R**^{**1**} Wasserstoff oder ein geradkettiger oder verzweigter C₁₋₂₀-Alkyl- oder C₂₋₂₀-Alkenylrest (mit oder ohne asymmetrische C-Atome), wobei
a) eine oder zwei nicht terminale CH₂-Gruppen unabhängig voneinander durch -O- oder -C(=O)- ersetzt sein können mit der Maßgabe, daß zwei benachbarte CH₂-Gruppen nicht gleich ersetzt sein können und/oder
b) eine CH₂-Gruppe durch -C≡C- ersetzt sein kann und/oder
c) eine CH₂-Gruppe durch -Si(CH₃)₂-, Cyclopropan-1,2-diyl, Cyclobutan-1,3-diyl, Cyclopentan-1,4-diyl, Bicyclo[1.1.1]pentan-1,3-diyl oder Cyclohexan-1,4-diyl ersetzt sein kann und/oder
d) ein oder mehrere H-Atome durch F und/oder CN ersetzt sein können;
e) im Falle eines verzweigten Alkylrestes mit asymmetrischen C-Atomen die asymmetrischen C-Atome -CH3, -OCH3, -CF3, F, CN und/oder Cl als Substituenten aufweisen oder in einen 3- bis 7-gliedrigen Ring eingebaut sind, worin auch eine oder zwei nicht benachbarte CH2-Gruppen durch -O- und eine zu diesen nicht benachbarte CH2-Gruppe durch -OC(=O)- ersetzt sein können;
**X** eine Einfachbindung, -O-, OC(=O)-, -C(=O)O- oder -OC(=O)O-
**Y** -OC(=O)- , -OCH₂-, -CH₂CH₂-
**A**^{**1**}**, A**^{**2**}**, A**^{**3**} sind unabhängig voneinander
Phenylen-1,4-diyl, gegebenenfalls ein- oder zweifach substituiert durch CN oder F, Phenylen-1,3-diyl, gegebenenfalls ein- oder zweifach substituiert durch CN oder F, Cyclohexan-1,4-diyl, wobei ein oder zwei H-Atome durch CN und/oder CH₃ und/oder F ersetzt sein können, 1-Cyclohexen-1,4-diyl, wobei ein H-Atom durch F ersetzt sein kann, 1-Alkyl-1-silacyclohexan-1,4-diyl, Pyridin-2,5-diyl, gegebenenfalls einfach substituiert durch F, Pyrimidin-2,5-diyl, gegebenenfalls einfach substituiert durch F, Cyclopentan-2,5-diyl, Thiophen-2,5-diyl;
**M**^{**1**}**, M**^{**2**} sind unabhängig voneinander ungerichtet
-OC(=O)-, -OCH₂-, -CH₂CH₂-, -OC(=O)CH₂CH₂-, -OCH₂CH₂CH₂-,
-C≡C-, -CH₂CH₂CH₂CH₂- oder eine Einfachbindung;
wenn **T** Furan-2,5-diyl oder Furan-2,4-diyl bedeutet, ist M¹ eine Einfachbindung;
**a, b** sind unabhängig voneinander gleich 0 oder 1.

2. Flüssigkristallmischung enthaltend mindestens eine Verbindung der Formel (I) gemäß Anspruch 1.

3. Flüssigkristallmischung nach Anspruch 2, **dadurch gekennzeichnet, daß** sie 0,01 bis 80 Gew.-% an einer oder mehreren Verbindungen der Formel (I) enthält.

4. Flüssigkristallmischung nach Anspruch 2 oder 3, **dadurch gekennzeichnet, daß** sie ferroelektrisch (chiral smektisch) ist.

5. Flüssigkristallmischung nach Anspruch 2 oder 3, **dadurch gekennzeichnet, daß** sie nematisch ist.

6. Ferroelektrische Schalt- und/oder Anzeigevorrichtung, enthaltend eine ferroelektrische Flüssigkristallmischung gemäß Anspruch 4.

7. Ferroelektrische Schalt- und/oder Anzeigevorrichtung gemäß Anspruch 6, **dadurch gekennzeichnet, daß** sie Aktivmatrixelemente enthält und die Flüssigkristallschicht eine monostabile Monodomäne ausbildet.

8. 5-Alkyl-4-fluor-thiophen-2-carbonsäuren der Formel (II) 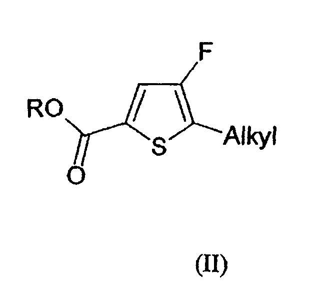 in der Alkyl ein geradkettiger oder verzweigter Alkylrest von 2 bis 16 C-Atomen, ausgenommen tert.-Butyl, und R Wasserstoff, Alkalimetall, Erdalkalimetall (1/2), ein geradkettiger oder verzweigter Alkylrest von 1 bis 16 Atomen, ausgenommen Methyl und tert.-Butyl, ist, oder entsprechende Säurehalogenide.

9. 5-Alkyl-3-fluor-thiophen-2-carbonsäuren der Formel (III) 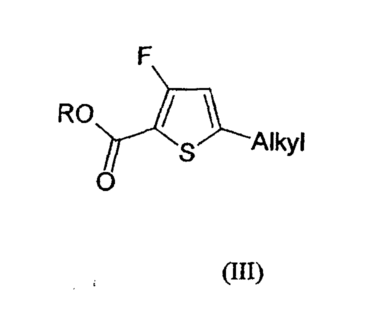 in der Alkyl ein geradkettiger oder verzweigter Alkylrest von 2 bis 16 C-Atomen und R Wasserstoff, Alkalimetall, Erdalkalimetall (1/2), ein geradkettiger oder verzweigter Alkylrest von 1 bis 16 Atomen ist, oder entsprechende Säurehalogenide.

10. Verwendung von Verbindungen der allgemeinen Formeln (II) und (III) gemäß Ansprüchen 8 und 9 fiir die Herstellung von Flüssigkristallen.

## Claims

1. A five-membered ring compound of the formula (I),
R¹-X-(A¹-M¹)ₐ-(A²-M²)_{b}-A³-Y-E (I)
where the symbols and indices have the following meanings:
**E** is a radical T-Z-R² containing a five-membered ring, where:
(i)
**T** is undirected and is
4-fluorothiophene-2,5-diyl, 3-fluorothiophene-2,5-diyl,
3-fluorothiophene-2,4-diyl or 5-fluorothiophene-2,4-diyl
**Z** is a single bond or -O-
**R**^{**2**} is hydrogen or a straight-chain or branched alkyl radical (with or without asymmetric carbon atoms) having 1 to 20 carbon atoms, where one nonterminal CH₂ group may be replaced by -O- or -OC(=O)- or -C(=O)O- and/or one or more H atoms may be replaced by F, with the provisos that
a) the -CH₂- group nearest to the thiophene cannot be replaced by -O- when Z is -O-
b) R² can only be hydrogen when Z is a single bond,
(ii)
**T** is furan-2,5-diyl or furan-2,4-diyl
**Z** is a single bond or -O-
**R**^{**2**} is a straight-chain or branched alkyl radical (with or without asymmetric carbon atoms) having 1 to 20 carbon atoms, where one nonterminal CH₂ group nonadjacent to furan may be replaced by -O- or -OC(=O)- or -C(=O)O- and/or one or more H atoms may be replaced by F,
**R**^{**1**} is hydrogen or a straight-chain or branched C₁₋₂₀-alkyl or C₂₋₂₀-alkenyl radical (with or without asymmetric carbon atoms), where
a) one or two nonterminal CH₂ groups may be replaced, independently of one another, by -O- or -C(=O)-, with the proviso that two adjacent CH₂ groups cannot be replaced in the same way, and/or
b) one CH₂ group may be replaced by -C≡C-, and/or
c) one CH₂ group may be replaced by -Si(CH₃)₂-, cyclopropane-1,2-diyl, cyclobutane-1,3-diyl, cyclopentane-1,4-diyl, bicyclo[1.1.1]pentane-1,3-diyl or cyclohexane-1,4-diyl, and/or
d) one or more H atoms may be replaced by F and/or CN,
e) in the case of a branched alkyl radical containing asymmetric carbon atoms, the asymmetric carbon atoms have -CH₃, -OCH₃, -CF₃, F, CN and/or Cl as substituents or
are incorporated into a 3- to 7-membered ring, in which, in addition, one or two non-adjacent CH₂ groups may be replaced by -O- and one CH₂ group non-adjacent to these groups may be replaced by -OC(=O)-;
**X** is a single bond, -O-, OC(=O)-, -C(=O)O- or -OC(=O)O-
**Y** is -OC(=O)-, -OCH₂-, -CH₂CH₂-
**A**^{**1**}**, A**^{**2**}**, A**^{**3**} are each, independently of one another,
phenylene-1,4-diyl, unsubstituted or monosubstituted or disubstituted by CN or F, phenylene-1,3-diyl, unsubstituted or monosubstituted or disubstituted by CN or F, cyclohexane-1,4-diyl, in which one or two H atoms may be replaced by CN and/or CH₃ and/or F, 1 -cyclohexene-1,4-diyl, in which one H atom may be replaced by F, 1-alkyl-1-silacyclohexane-1,4-diyl, pyridine-2,5-diyl, unsubstituted or monosubstituted by F, pyrimidine-2,5-diyl, unsubstituted or monosubstituted by F, cyclopentane-2,5-diyl or thiophene-2,5-diyl;
**M**^{**1**}**, M**^{**2**} are undirected and are each, independently of one another,
-OC(=O)-, -OCH₂-, -CH₂CH₂-, -OC(=O)CH₂CH₂-, -OCH₂CH₂CH₂-, -C≡C-, -CH₂CH₂CH₂CH₂- or a single bond;
when T is furan-2,5-diyl or furan-2,4-diyl, M¹ is a single bond;
**a, b** are each, independently of one another, 0 or 1.

2. A liquid-crystal mixture comprising at least one compound of the formula (I) as claimed in claim 1.

3. A liquid-crystal mixture as claimed in claim 2, which comprises from 0.01 to 80% by weight of one or more compounds of the formula (I).

4. A liquid-crystal mixture as claimed in claim 2 or 3, which is ferroelectric (chiral smectic).

5. A liquid-crystal mixture as claimed in claim 2 or 3, which is nematic.

6. A ferroelectric switching and/or display device, which contains a ferroelectric liquid-crystal mixture as claimed in claim 4.

7. A ferroelectric switching and/or display device as claimed in claim 6, which contains active matrix elements and wherein the liquid-crystal layer forms a monostable monodomain.

8. A 5-alkyl-4-fluoro-thiophene-2-carboxylic acid of the formula (II) 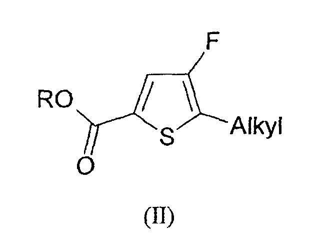 in which alkyl is a straight-chain or branched alkyl radical of 2 to 16 carbon atoms with the exception of tert.-butyl, and R is hydrogen, alkali metal, alkaline earth metal (1/2), a straight-chain or branched alkyl radical of 1 to 16 atoms with the exception of methyl and tert-butyl, or a corresponding acid halide.

9. A 5-alkyl-3-fluoro-thiophene-2-carboxylic acid of the formula (III) 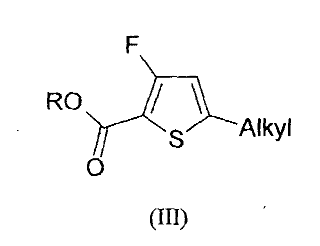 in which alkyl is a straight-chain or branched alkyl radical of 2 to 16 carbon atoms and R is hydrogen, alkali metal, alkaline earth metal (1/2), a straight-chain or branched alkyl radical of 1 to 16 atoms or a corresponding acid halide.

10. The use of compounds of the general formulae (II) and (III) as claimed in claims 8 and 9 for preparing liquid crystals.

## Revendications

1. Composés à noyaux pentagonaux de formule (I)
R¹-X-(A¹-M¹)ₐ-(A²-M²)_{b}-A³-Y-E (I)
les symboles et les indices possédant les significations suivantes :
E représente un reste T-Z-R² présentant un noyau pentagonal possédant les significations suivantes :
(i)
T non orienté représente
4-fluorothiophène-2,5-diyle, 3-fluorothiophène-2,5-diyle, 3-fluorothiophène-2,4-diyle ou 5-fluorothiophène-2,4-diyle
Z représente une simple liaison ou -O-
R² représente un atome d'hydrogène ou un reste alkyle linéaire ou ramifié (avec ou sans atomes de C asymétriques) présentant 1 à 20 atomes de carbone, un groupe -CH₂- non terminal pouvant être remplacé par -O- ou -OC(=O)- ou -C(=O)O- et/ou un ou plusieurs atomes de H pouvant être remplacés par un atome de F à condition que
a) le groupe -CH₂- le plus proche du thiophène ne puisse pas être remplacé par -O- lorsque Z représente -O-,
b) R² ne puisse représenter un atome d'hydrogène que si Z est une simple liaison,
(ii)
T représente un groupe furanne-2,5-diyle ou furanne-2,4-diyle,
Z est une simple liaison ou -O-
R² représente un reste alkyle linéaire ou ramifié (avec ou sans atomes de carbone asymétriques) présentant 1 à 20 atomes de carbone, un groupe -CH₂- non terminal, non adjacent au groupe furanne, pouvant être remplacé par -O- ou -OC(=O)- ou -C(=O)O- et/ou un ou plusieurs atomes de H pouvant être remplacés par des atomes de F,
R¹ représente un atome d'hydrogène ou un reste alkyle en C₁-C₂₀ ou alcényle en C₂-C₂₀ linéaire ou ramifié (avec ou sans atomes de carbone asymétriques),
a) un ou deux groupes -CH₂- non terminaux peuvent être remplacés indépendamment l'un de l'autre par -O- ou -C(=O)- à condition que deux groupes -CH₂- voisins ne puissent pas être remplacés de la même manière et/ou
b) un groupe -CH₂- peut être remplacé par un groupe -C≡C- et/ou
c) un groupe -CH₂- peut être remplacé par des groupes -Si(CH₃)₂-, cyclopropane-1,2-diyle, cyclobutane-1,3-diyle, cyclopentane-1,4-diyle, bicyclo[1.1.1]pentane-1,3-diyle ou cyclohexane-1,4-diyle et/ou
d) un ou plusieurs atomes de H pouvant être remplacés par un atome de F et/ou un groupe CN ;
e) dans le cas d'un reste alkyle ramifié avec des atomes de C asymétriques, les atomes de carbone asymétriques peuvent présenter des groupes -CH₃, -OCH₃, -CF₃, F, CN et/ou Cl en tant que substituants ou sont incorporés dans un cycle de 3 à 7 chaînons, où aussi un ou deux groupes -CH₂- non adjacents peuvent être remplacés par -O- et un groupe -CH₂- non adjacent à ceux-ci peut aussi être remplacé par un groupe -OC(=O)- ;
X représente une simple liaison, des groupes -O-, -OC(=O)-, -C(=O)O- ou -OC(=O)O-
Y représente des groupes -OC(=O)-, -OCH₂-, -CH₂CH₂-,
A¹, A², A³ représentent indépendamment les uns des autres des groupes
phénylène-1,4-diyle, éventuellement substitué une ou deux fois par des substituants CN ou F, phe-nylène-1,3-diyle, éventuellement substitué une ou deux fois par des substituants CN ou F, cyclohexane-1,4-diyle, un ou deux atomes de H pouvant être remplacés par des groupes CN et/ou CH₃ et/ou F, 1-cyclohexène-1,4-diyle, un atome de H pouvant être remplacé par un atome de F, 1-alkyl-1-silacyclohexane-1,4-diyle, pyridine-2,5-diyle, éventuellement substitué une fois par un atome de F, pyrimidine-2,5-diyle, éventuellement substitué une fois par un atome de F, cyclopentane-2,5-diyle, thiophène-2,5-diyle ;
M¹, M² représentent non orientés indépendamment l'un de l'autre des groupes
-OC(=O)-, -OCH₂, -CH₂CH₂-, -OC(=O)CH₂CH₂-, -OCH₂CH₂CH₂ -, -C≡C-, -CH₂CH₂CH₂CH₂- ou une simple liaison ;
lorsque T représente un groupe furanne-2,5-diyle ou furanne-2,4-diyle, M¹ est une simple liaison ;
a, b valent indépendamment 0 ou 1.

2. Mélange de cristaux liquides renfermant au moins un composé de formule (I) selon la revendication 1.

3. Mélange de cristaux liquides selon la revendication 2, **caractérisé en ce qu'**il présente une teneur de 0,01 à 80 % en poids en un ou plusieurs composés de formule (I).

4. Mélange de cristaux liquides selon la revendication 2 ou 3, **caractérisé en ce qu'**il est ferroélectrique (chiral smectique).

5. Mélange de cristaux liquides selon la revendication 2 ou 3, **caractérisé en ce qu'**il est nématique.

6. Dispositif de commutation et/ou d'affichage ferroélectrique renfermant un mélange de cristaux liquides ferroélectrique selon la revendication 4.

7. Dispositif de commutation et/ou d'affichage ferroélectrique selon la revendication 6, **caractérisé en ce qu'**il renferme des éléments de matrice actifs et la couche à cristal liquide forme un monodomaine monostable.

8. Acide 5-alkyl-4-fluro-thiophène-2-carboxylique de formule (I) 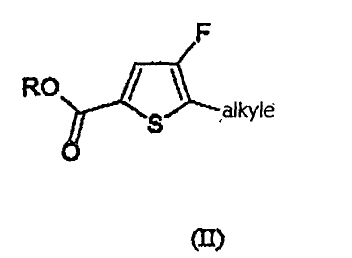 dans laquelle le groupe alkyle représente un reste alkyle linéaire ou ramifié présentant 2 à 16 atomes de carbone à l'exception du groupe tert-butyle, et R représente des atomes d'hydrogène, de métal alcalin, de métal alcalino-terreux (1/2), un reste alkyle linéaire ou ramifié présentant 1 à 16 atomes de carbone, à l'exception de groupes méthyle et tert-butyle, ou des halogénures correspondants.

9. Acide 5-alkyl-3-fluro-thiophène-2-carboxylique de formule (III) 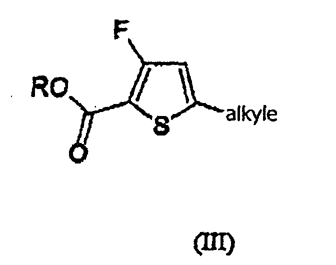 dans laquelle le groupe alkyle représente un reste alkyle linéaire ou ramifié présentant 2 à 16 atomes de carbone et R représente des atomes d'hydrogène, de métal alcalin, de métal alcalino-terreux (1/2), un reste alkyle linéaire ou ramifié présentant 1 à 16 atomes de carbone ou des halogénures correspondants.

10. Utilisation de composés de formules générales (II) et (III) selon les revendications 8 et 9 pour la préparation de cristaux liquides.
